(19) Europäisches Patentamt
European Patent Office
Office européen des brevets

(11) **EP 3 448 550 B1**

(12) **EUROPÄISCHE PATENTSCHRIFT**

(45) Veröffentlichungstag und Bekanntmachung des Hinweises auf die Patenterteilung:
**17.08.2022   Patentblatt 2022/33**

(21) Anmeldenummer: **17723005.9**

(22) Anmeldetag: **21.04.2017**

(51) Internationale Patentklassifikation (IPC):
**B01D 61/16** $^{(2006.01)}$     **A61L 2/00** $^{(2006.01)}$
**B01D 65/10** $^{(2006.01)}$     **B01D 61/58** $^{(2006.01)}$
**B01D 71/56** $^{(2006.01)}$     **B01D 71/68** $^{(2006.01)}$

(52) Gemeinsame Patentklassifikation (CPC):
(C-Sets verfügbar)
**B01D 61/16; B01D 65/10;** B01D 61/58;
B01D 71/56; B01D 71/68; B01D 2311/04;
B01D 2317/025                              (Forts.)

(86) Internationale Anmeldenummer:
**PCT/EP2017/000513**

(87) Internationale Veröffentlichungsnummer:
**WO 2017/186346 (02.11.2017 Gazette 2017/44)**

(54) **VERFAHREN ZUR BESTIMMUNG DES LOGARITHMISCHEN REDUKTIONSWERTES LRV EINES GRÖSSENAUSSCHLUSSFILTERS**

METHOD FOR DETERMINING THE LOGARITHMIC REDUCTION VALUE LRV OF A SIZE EXCLUSION FILTER

PROCÉDÉ POUR DÉTERMINER LA VALEUR DE RÉDUCTION LOGARITHMIQUE (LRV) D'UN FILTRE À EXCLUSION DE TAILLE

(84) Benannte Vertragsstaaten:
**AL AT BE BG CH CY CZ DK EE ES FI FR GB GR HR HU IE IS IT LI LT LU LV MC MK MT NL NO PL PT RO RS SE SI SK SM TR**

(30) Priorität: **26.04.2016   DE 102016005049**

(43) Veröffentlichungstag der Anmeldung:
**06.03.2019   Patentblatt 2019/10**

(73) Patentinhaber: **Sartorius Stedim Biotech GmbH**
**37079 Göttingen (DE)**

(72) Erfinder:
• **HANSMANN, Björn**
**37077 Göttingen (DE)**
• **PEIKER, Marcus**
**88487 Mietingen (DE)**

• **THOM, Volkmar**
**37083 Göttingen (DE)**

(74) Vertreter: **Novagraaf Group**
**Chemin de l'Echo 3**
**1213 Onex/ Geneva (CH)**

(56) Entgegenhaltungen:
**WO-A1-2007/039069     WO-A1-2010/098867**
**US-A1- 2015 013 434**

(52) Gemeinsame Patentklassifikation (CPC): (Forts.)

C-Sets
B01D 2311/04, B01D 2311/2649

**Beschreibung**

Die vorliegende Erfindung betrifft ein Verfahren zur Bestimmung des logarithmischen Reduktionswertes LRV eines Größenausschlussfilters für ein zu klärendes Partikel einer Prozesslösung, wobei der Größenausschlussfilter durch einen seriell vorgeschalteten Prozessadsorber vor einer verblockenden adsorbierenden Spezies, enthalten in der Prozesslösung, geschützt wird.

Die Ergebnisse aus Virusabreicherungsstudien sind in der (bio-)pharmazeutischen Industrie ein integraler Bestandteil des Zulassungsdossiers und somit entscheidend für die Beweisführung für die Arzneimittelsicherheit von Produkten, die in Säugetierzellen hergestellt werden. Die Abreicherung von Viren erfolgt dabei beispielsweise aus Proteinlösungen, die sowohl Proteinmonomere als auch Proteinaggregate enthalten. Sowohl in den Virusabreicherungsstudien als auch im Produktionsmaßstab wird gewöhnlich eine Kombination aus einem Vorfilter (Prozessadsorber), der sowohl adsorptiv die Proteinaggregate als auch nach dem Größenausschlussprinzip die Viren zurückhält, und einem Virusfilter, der lediglich nach dem Größenausschlussprinzip die Viren zurückhält, verwendet. Bei einer Virusabreicherungsstudie kann durch diese (unerwünschte) Virusrückhaltung durch den Vorfilter die durch den Virusfilter erreichte Virusrückhaltung höher erscheinen als sie tatsächlich wäre, wenn man die Virusrückhaltung des Virusfilters alleine untersuchen würde. Somit war es bisher im Stand der Technik nicht möglich, präzise zwischen der Virusrückhaltung durch den Vorfilter und den Virusfilter zu unterscheiden.

Virusabreicherungsstudien werden zum größten Teil in spezialisierten Firmen, sogenannten Auftragslabors, durchgeführt. Hierzu ist es im Regelfall notwendig, die Ausgangsmaterialien für die einzelnen Prozessstufen an die Auftragslabors zu verschicken. In vielen Fällen erfolgt der Transport tiefgefroren (-20°C / -70°C). In den Fällen, in denen ein Einfrieren aufgrund der Produktstabilität (Aggregatbildung) nicht möglich ist, erfolgt der Transport gekühlt (2 bis 8°C).

Das Verfahren "Einfrieren/Auftauen" neigt dazu, in der Prozesslösung die Bildung von Aggregaten des Zielproduktes oder aber auch von Nebenprodukten zu begünstigen oder sogar zu verstärken. Dies sind im Umkehrschluss Artefakte, die im GMP-Prozess ("Good-Manufacturing-Practice"-Prozess) durch die direkte Weiterverarbeitung der Prozessintermediate nicht zu finden sind. Um die aufgetaute Prozesslösung als "repräsentativ" in der Virusstudie einsetzen zu können, müssen diese Aggregate durch eine Vorfiltration über einen Vorfilter, den sogenannten Prozessadsorber, abgereichert werden.

Im Prozessschritt "Virusfiltration" ist die Bildung von Aggregaten auch im eigentlichen Prozess unerwünscht, da die Aggregate die engporigen Virusfiltermembranen (Größenausschlussmembranen) verblocken können und somit die Leistungsfähigkeit (Flussrate/Filtrationskapazität/Standzeit) des (Größenausschluss-)Filters stark beeinträchtigt wird.

Eine Vorfiltration über einen Prozessadsorber während der Virusstudien ist demnach entweder im Rahmen der Prozessführung bei "schwer zu filtrierenden Lösungen" oder aber durch den Umstand der Bereinigung nach "Einfrieren/Auftauen" unumgänglich.

Für die Fälle, in denen die Vorfiltration dazu dient, die Aggregate abzureichern, die sich im Rahmen des "Einfrierens/Auftauens" gebildet haben, ist die Gefahr der Verblockung des eigentlichen Virusfilters (Größenausschlussfilters) bereits durch eine "off-line" durchgeführte Vorfiltration gebannt, wenn in diesen Fällen die Prozesslösung von sich aus nicht dazu neigt, Aggregate nachzubilden.

Das Vorgehen bei "schwer zu filtrierenden Lösungen" jedoch erfordert, dass die Vorfiltration auch im Rahmen der Virusstudie "in-line" erfolgt, wenn die Prozesslösung nach einer "off-line" durchgeführten Vorfiltration im Rahmen einer Virusstudie erneut genug Zeit hat, Aggregate zu bilden. Diese "in-line"-Vorfiltration ist allerdings mit der etablierten Vorgehensweise in Virusstudien in der aktuellen Form nicht kompatibel.

Als Vorfilter (Prozessadsorber) werden üblicherweise adsorbierende Materialien verwendet. Derartige adsorbierende Materialien werden in verschiedensten Bereichen der Industrie verwendet, um Trennungen aufgrund spezifischer oder unspezifischer Adsorption zu erreichen. Adsorption ist dabei ein Oberflächenphänomen. Oft werden als adsorptive Vorfilter poröse Materialien verwendet, da sie auf einer kleinen, kompakten äußeren Form eine große, für bindende Verunreinigung zugängliche innere Oberfläche bereitstellen. Diese porösen Materialien sind unter anderem gekennzeichnet durch eine Porengröße, welche sich auf die Trenneigenschaft des Materials aufgrund von Größenausschluss auswirkt. Die Schwierigkeit besteht darin, einerseits eine große Oberfläche für die Bindung bereitzustellen und eine kompakte äußere Form zu erhalten, aber andererseits die Porengröße so zu wählen, dass nachweislich kein signifikanter Größenausschluss von Viren bei der Verwendung in einer Virusabreicherungsstudie am Vorfilter zu beobachten ist. Mit einer Porengröße, die für den Produktionsmaßstab und gleichzeitig für die Virusabreicherungsstudie verwendet wird, ist das nur unzureichend möglich.

Größenausschlusseigenschaften sind im Rahmen der vorliegenden Erfindung alle Eigenschaften, die einer mechanischen Trennung aufgrund der Größe, also Längen- oder Volumenausdehnung, von Partikeln dienen. Für Formkörper oder Vorrichtungen, die Größenausschlusseigenschaften besitzen, ist es charakteristisch, dass eine Partikeltrenngrenze anhand von Probepartikeln definiert werden kann. Die Partikeltrenngrenze ist definiert durch den Durchmesser von Probepartikeln, die zu einem definierten Anteil der Konzentration oder Zahl zurückgehalten werden. Eine typische Definition einer Partikeltrenngrenze ist der 90% "Cut-off". Je nach benötigter Probengröße werden Formkörper einer möglichst engen Größenverteilung oder geeignete Moleküle für die Bestimmung der Partikeltrenngrenze verwendet. Bei molekularen Partikeln wird alternativ zu einem Durchmesser das Molekulargewicht verwendet. WO 2007/039069 offenbart eine Abtrennung von gröberen organischen Verunreinigungen in einem Vorfilter und in einem stromabwärts angeordneten Filter die Abtrennung von Viren.

Eine Beschreibung, wie im Stand der Technik adsorptive Vorfilter verwendet werden und was unter adsorptiven Vorfiltern (Prozessadsorbern) im Sinne der vorliegenden Anmeldung zu verstehen ist, findet sich beispielsweise in der DE 10 2011 105 525 A1. Dort wird offenbart, dass ein Formkörper aus einem Polyamid in einem Verfahren angewendet werden kann, das Biopolymere und Viren aus einem Fluid entfernt. Ein anderes Verfahren, offenbart in US 7,118,675 B2, beschreibt die Verwendung von geladenen und/oder modifizierten Membranen oder Tiefenfiltermaterialien mit Diatomeenerde, die für die Entfernung von Aggregaten vor der virusabreichernden Filtration verwendet werden. Keine der vorstehenden Anmeldungen thematisiert jedoch die Verwendung in der Abreicherungsstudie und behebt nicht das Problem, das bei der Verwendung dieser Verfahren durch die nicht näher definierte Virusabreicherung der Vorfilter entsteht.

Das Problem, dass durch Verunreinigungen der Lösung in einer Virusabreicherungsstudie entsteht, nämlich, dass die Skalierung der Filterflächen systematisch zwischen der Virusabreicherungsstudie und dem Produktionsmaßstab abweicht, wird in US 2012/088228 A1 beschrieben. Hier wird offenbart, dass Verunreinigungen aus der Virus-Stammlösung minimiert und die Konzentration der Stammlösung erhöht werden können, indem eine Ultrazentrifugation und ein Adsorptionsschritt an einem Kationenaustauscher oder allgemein ein chromatographischer Reinigungsschritt durchgeführt wird. Die Materialien, die dort zur chromatographischen Reinigung beschrieben werden, entsprechen weitgehend den Materialien, die auch in der US 7,118,675 B2 genannt sind. Zusätzlich werden explizit auch chromatographische Säulen als Medien genannt. Bei der Anwendung des Verfahrens der US 2012/088228 A1 wird jedoch nicht das Problem gelöst, dass die Verunreinigungen auch aus der Prozesslösung stammen können und im Produktionsmaßstab durch adsorbierende Vorfilter entfernt werden, deren Verwendung in der Virusabreicherungstudie nicht "in-line" möglich ist.

Eine Virusabreicherungsstudie mit einer "in-line" erfolgenden Vorfiltration kann beispielsweise folgendermaßen durchgeführt werden:

- optional: Auftauen der (tief-)gefrorenen Prozesslösung
- Aliquotierung der Prozesslösung
- Zugabe der Virus-Stammlösung in einem vordefinierten Verhältnis ("spike ratio" in [% (v/v)])
- Je nach Typ/Größe des zugegebenen Virus wird eine prozessunabhängige Vorfiltration durchgeführt, um dafür zu sorgen, dass die zugegebenen Viruspartikel "mono-dispers", also ohne Virus-Aggregate, vorliegen. Beispielsweise wird für das Modellvirus MuLV (Murines Leukämievirus, 80 - 120 nm) eine Vorfiltration mit 0.45 $\mu$m Porengröße mit einem Größenausschlussfilter durchgeführt, für Vertreter der Parvoviren (z.B. PPV oder MVM; 18 - 24 nm) wird ein 0.1 $\mu$m Größenausschlussfilter verwendet.
- Abnahme der Probe "Load" durch Mitarbeiter des Auftragslabors, um den Startwert der Viruskonzentration bestimmen zu können.
- Durchführung der Virusfiltration mit einem "in-line" eingebauten Vorfilter (Prozessadsorber) vor dem eigentlichen Virusfilter (Größenausschlussfilter).
- Abnahme der Probe "Filtrat" durch Mitarbeiter des Auftragslabors, um den Endwert der Viruskonzentration nach der Virusfiltration bestimmen zu können.

Die regulatorischen Behörden (z.B. ICH Guideline Q5A (R1), Seite 8, 2. Abschnitt) fordern, dass die Wirkmechanismen der einzelnen virusabreichernden Prozessschritte bekannt und eindeutig identifiziert sein müssen. Die gleichzeitige Verwendung mehrerer Schritte zur Virusabreicherung beispielsweise durch eine Kombination aus Adsorption und Größenausschluss ist unerwünscht, da in diesem Fall die Abreicherung nicht wie regulatorisch gefordert auf ein definiertes Wirkprinzip zurückgeführt werden kann. Üblicherweise erfolgt die Virusabreicherung bei der Verwendung eines isolierten Größenausschlussfilters über den Mechanismus des Größenausschlusses. Im Fall des vorstehend beschriebenen Verfahrens ist der Mechanismus jedoch unklar, da sowohl Adsorption als auch Größenausschluss hier einen Beitrag leisten können.

Des Weiteren werden im gesamten Prozess der Herstellung biologischer Pharmazeutika orthogonale Methoden zur Abreicherung gefordert. Orthogonal bezieht sich hierbei auf das zugrundeliegende Wirkprinzip der Methode zur Abreicherung. So könnte zwar auch eine Abreicherung durch die serielle Durchführung der gleichen Methode gezeigt werden, aber die Summierung der gezeigten Abreicherungen würde den tatsächlichen Wert wahrscheinlich überschätzen.

Diese Annahme basiert darauf, dass eine Population an Viren bezüglich ihrer Eigenschaften, die für die Abreicherung mit einer speziellen Methode entscheidend ist, durchaus heterogen sein kann. So kann beispielsweise die Affinität der Viren zu einem Adsorber sogar innerhalb der gleichen Virus-Spezies so heterogen sein, dass die Abreicherung zu einer Selektion führt. Diese vorselektierte Virus-Population wird sich in einem weiteren Adsorptionsschritt anders verhalten als beim ersten Schritt.

Eine zentrale Anforderung an Virusabreicherungsstudien ist die Skalierbarkeit. Zwar ist es technisch nicht möglich, Studien im tatsächlichen Produktionsmaßstab durchzuführen, jedoch soll dieser in den Studien möglichst genau abgebildet werden. Die Abbildung des Produktionsmaßstabs bei der Verwendung adsorptiver Vorfilter (Prozessadsorber), die im Produktionsmaßstab erfolgreich und vorteilhaft eingesetzt werden, ist in den bisher bekannten Verfahren nur eingeschränkt möglich. Insbesondere besteht im bisher bekannten Stand der Technik das Problem, dass bei der Verwendung von vorgeschalteten Prozessadsorbern häufig nicht zwischen einer Trennung aufgrund der adsorbierenden Eigenschaften und aufgrund der Größenausschlusseigenschaften unterschieden werden kann.

Demzufolge liegt der vorliegenden Erfindung die Aufgabe zugrunde, ein Verfahren zur Bestimmung des logarithmischen Reduktionswertes (LRV) eines Größenausschlussfilters, der durch einen seriell vorgeschalteten Prozessadsorber vor einer verblockenden adsorbierenden Spezies geschützt wird, bereitzustellen, welches es ermöglicht, die beiden Trennmechanismen "Adsorption" und "Größenausschluss" voneinander zu separieren, um so in einer Virusabreicherungsstudie im Labormaßstab eine realistischere Abbildung des Produktionsmaßstabs erzielen zu können.

Gemäß der vorliegenden Erfindung wird daher ein Verfahren zur Bestimmung des logarithmischen Reduktionswertes $LRV_{Größenausschlussfilter}$ eines Größenausschlussfilters G für ein zu klärendes Partikel einer Prozesslösung bereitgestellt, wobei der Größenausschlussfilter G durch einen seriell vorgeschalteten Prozessadsorber P vor einer verblockenden adsorbierenden Spezies, enthalten in der Prozesslösung, geschützt wird und wobei durch den Prozessadsorber P das zu klärende Partikel mit einem $LRV_{Prozessabsorber}$ von 0,5 oder mehr zurückgehalten wird, umfassend die Schritte:

(a) Bereitstellen eines Testsystems, umfassend

einen Größenausschlussfilter G, dessen $LRV_{Größenausschlussfilter}$ zu bestimmen ist, und einen dem Größenausschlussfilter G seriell vorgeschalteten Testadsorber T, welcher aus einem gleichartigen Material wie der Prozessadsorber P besteht und durch den das zu klärende Partikel mit einem bekannten $LRV_{Testadsorber}$ von 2 oder weniger zurückgehalten wird, wobei $LRV_{Testadsorber} < LRV_{Prozessadsorber}$ gilt;

(b) Bestimmung von $LRV_{Testsystem}$ des Testsystems für das zu klärende Partikel; und
(c) Berechnung von $LRV_{Größenausschlussfilter}$ durch Subtraktion des $LRV_{Testadsorber}$ von $LRV_{Testsystem}$.

Gemäß der vorliegenden Erfindung ist der logarithmische Reduktionswert LRV (*"logarithmic reduction value"*) definiert als der Logarithmus des Quotienten der Konzentration "$C_{feed}$" einer abzutrennenden Substanz in einer Prozesslösung ("Speiselösung") vor Durchlauf eines Filters und der Konzentration "$C_{filtrate}$" einer abzutrennenden Substanz

$$LRV = \log\frac{C_{feed}}{C_{filtrate}}$$

in der Prozesslösung nach Durchlauf des Filters (also im Filtrat), d.h.

Erfindungsgemäß unterliegt der Begriff "Prozesslösung" keiner besonderen Einschränkung und bezeichnet insbesondere eine wässrige Lösung (beispielsweise eine Proteinlösung), die eine verblockende adsorbierende Spezies (beispielsweise Biopolymere und/oder Polypeptide), ein zu entfernendes Partikel (beispielsweise Viren) und ein Zielmolekül (beispielsweise monoklonale oder polyklonale Antikörper, ein rekombinantes Protein oder Produkte aus Blut, Blutplasma wie Albumin, Immunoglobuline, Gerinnungsfaktoren, andere Proteine oder Polypeptide, die in Blut, Blutplasma oder im Inneren von eukaryotischen Zellen vorhanden sind) umfasst.

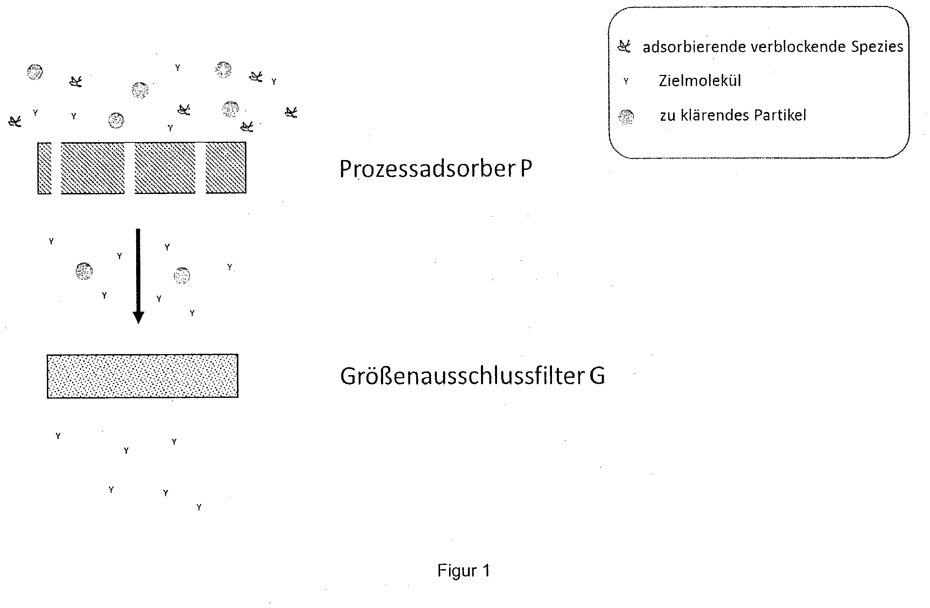

Das erfindungsgemäße Verfahren ermöglicht die Bestimmung des $LRV_{Größenausschlussfilter}$ eines Größenausschlussfilters G für ein zu klärendes Partikel einer Prozesslösung in einem Filtrationssystem, das den Größenausschlussfilter G und einen seriell vorgeschalteten Prozessadsorber P umfasst. Der seriell vorgeschaltete Prozessadsorber ist in diesem Filtrationssystem nötig, um den Größenausschlussfilter G vor der verblockenden adsorbierenden Spezies, enthalten in der Prozesslösung, zu schützen. Der Prozessadsorber P hält dabei bereits selbst das zu klärende Partikel mit einem $LRV_{Prozessabsorber}$ von 0,5 oder mehr zurück, d.h. mindestens 68,4% der zu klärenden Partikel werden bereits durch den Prozessadsorber P aus der Prozesslösung entfernt. Vorteilhafterweise kann über das erfindungsgemäße Verfahren der $LRV_{Größenausschlussfilter}$ des Größenausschlussfilters G für das zu entfernendes Partikel bestimmt werden, obwohl im regulären Betrieb (d.h. im Produktionsgroßmaßstab) bereits der Prozessadsorber P einen Teil der zu klärenden Spezies aus der Prozesslösung entfernt (siehe Figur 1).

Erfindungsgemäß wird die Vorfiltration durch den Prozessadsorber bzw. den Testadsorber "in-line" durchgeführt, so dass sie der Richtlinie genügt, dass der Mechanismus der Virusabreicherung eindeutig identifiziert ist.

Im Schritt (a) des erfindungsgemäßen Verfahrens wird ein Testsystem, umfassend den Größenausschlussfilter G, dessen LRV$_{Größenausschlussfilter}$ zu bestimmen ist, und einen dem Größenausschlussfilter G seriell vorgeschalteten Testadsorber T bereitgestellt. Der Testadsorber T besteht dabei aus einem gleichartigen Material wie der Prozessadsorber P, d.h. Testadsorber T und Prozessadsorber P bestehen aus dem gleichen Basismaterial (wie z.B. Polyethersulfon (PES), Polyamid (PA), Polypropylen (PP) etc.).

Erfindungsgemäß wird durch den Testadsorber T das zu klärende Partikel mit einem bekannten LRV$_{Testadsorber}$ von 2 oder weniger zurückgehalten, wobei LRV$_{Testadsorber}$ < LRV$_{Prozessadsorber}$ gilt, d.h. der Testadsorber T hält weniger zu klärende Partikel zurück als der Prozessadsorber P.

Der genaue LRV$_{Testadsorber}$ des Testadsorbers T ist für die konkreten Prozessbedingungen des erfindungsgemäßen Verfahrens bekannt (beispielsweise aus Angaben des Herstellers des Testadsorbers), oder wird in einem optionalen Schritt vor dem Schritt (a) für die konkreten Prozessbedingungen, d.h. in Abhängigkeit des zu klärenden Partikels, des pH-Werts und der Salzkonzentration der zu klärenden Lösung usw., bestimmt. Dies erfolgt beispielsweise dadurch, dass die Konzentration des zu klärenden Partikels in der Prozesslösung vor Durchlauf des Testadsorbers ("c$_{feed}$") und die Konzentration des zu klärenden Partikels in der Prozesslösung nach Durchlauf des Testadsorbers ("c$_{filtrate}$") bestimmt und aus dem Quotienten dieser Größen der Logarithmus berechnet wird, wie vorstehend beschrieben. Die vorstehend beschriebene Bestimmung des LRV$_{Testadsorber}$ erfolgt vorzugsweise an einem Testadsorber T, der noch nicht in das Testsystem des erfindungsgemäßen Verfahrens eingebaut ist.

Demzufolge umfasst das erfindungsgemäße Verfahren optional vor dem Schritt (a) den Schritt (a') Bestimmung des LRV$_{Testadsorber}$ des Testadsorbers T für das zu klärende Partikel der Prozesslösung, wobei der Testadsorber T im Schritt (a') entweder baugleich zu (z.B. gleiche Charge) oder identisch (Weiterverwendung z.B. nach Regeneration) dem Testadsorber T des Testsystems im Schritt (a) des erfindungsgemäßen Verfahrens ist.

Im Schritt (b) des erfindungsgemäßen Verfahrens wird der LRV$_{Testsystem}$ des Testsystems für das zu klärende Partikel bestimmt.

Im Schritt (c) des erfindungsgemäßen Verfahrens wird durch Subtraktion des LRV$_{Testadsorber}$ von LRV$_{Testsystem}$ der LRV$_{Größenausschlussfilter}$ berechnet, d.h. LRV$_{Größenausschlussfilter}$ = LRV$_{Testsystem}$ - LRV$_{Testadsorber}$.

Die vorliegende Erfindung beruht auf der Erkenntnis, dass der LRV$_{Größenausschlussfilter}$ eines Größenausschlussfilters G für ein zu klärendes Partikel einer Prozesslösung in einem Filtrationssystem im Produktionsmaßstab, bei dem ein seriell vorgeschalteter Prozessadsorber P nötig ist, um den Größenausschlussfilter G vor einer verblockenden adsorbierenden Spezies zu schützen, bestimmt werden kann, indem Versuche an einem Testsystem mit dem gleichen Größenausschlussfilter G (d.h. mit gleichem "Cut-off"-Wert, jedoch in geringerer Dimensionierung) durchgeführt werden, der ebenso von einem Vorfilter (Testadsorber T) in gleicher Weise vor der verblockenden adsorbierenden Spezies geschützt wird, wobei der Testadsorber T des Testsystems im Labormaßstab aufgrund größerer Poren jedoch eine ausreichend große Menge (LRVTestadsorber ≤ 2) an zu klärendem Partikel passieren lässt.

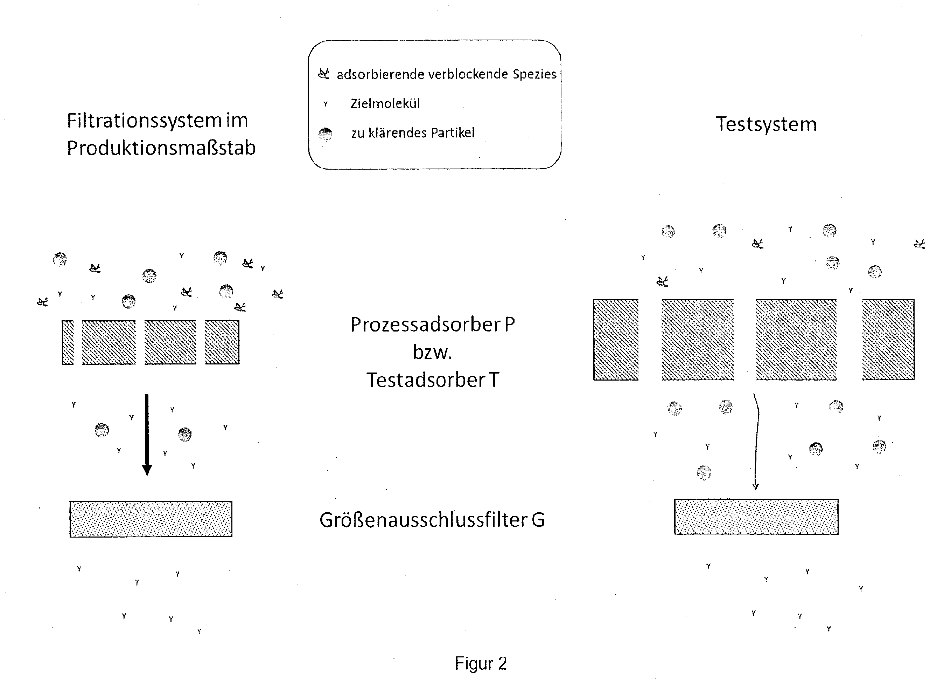

Je größer die Menge an zu klärendem Partikel ist, welche der Testadsorber T des Testsystems ungehindert passieren lässt, desto zuverlässiger kann der logarithmische Reduktionswertes LRV$_{Größenausschlussfilter}$ des Größenausschlussfilters G für das zu klärendes Partikel der Prozesslösung bestimmt werden. Demzufolge weist gemäß einer bevorzugten Ausführungsform der vorliegenden Erfindung der Testadsorber T einen LRV$_{Testadsorber}$ von 1,5 oder weniger auf, mehr bevorzugt 1,0 oder weniger, und noch mehr bevorzugt 0,5 oder weniger. Am meisten bevorzugt weist der Testadsorber T einen LRV$_{Testadsorber}$ von gegen "0" gehend bzw. exakt "0" auf, da in diesem Fall (annähernd) alle zu klärenden Partikel der Prozesslösung den Testadsorber ungehindert passieren, wobei der Größenausschlussfilter G weiterhin vor der verblockenden adsorbierenden Spezies geschützt wird (siehe Figur 2).

Adsorber, d.h. Prozessadsorber und Testadsorber, die im Verfahren der vorliegenden Erfindung verwendet werden können, sind allgemein Materialien, die an ihrer äußeren und inneren Oberfläche Stoffe durch spezifische chemische Gruppen, die auf der Oberfläche angebracht sind (sogenannte Liganden), chemisch oder physikalisch binden, also immobilisieren können. Beispiele für diese Adsorber, die über Liganden verfügen, sind die geladenen und/oder modifizierten Membranen, wie sie in US 7,118,675 B2 beschrieben sind, darüber hinaus typische chromatographische Medien wie Gele, Monolithe, Beads ("Kügelchen") und andere Säulenmaterialien aus polymeren Trägermaterialien oder anorganischen Trägermaterialien, wie beispielsweise Silicagel, Kieselgur oder Aluminiumoxid, auf die Liganden aufgebracht sind oder die durch ihre Herstellung Liganden tragen. Darüber hinaus werden mit den Adsorbern dieser Erfindung auch Materialien bezeichnet, die sich in ihren Eigenschaften an der äußeren beziehungsweise inneren Oberfläche nicht von den Eigenschaften im Volumen unterscheiden (also keine Liganden enthalten) und ebenfalls über die Eigenschaft verfügen, Stoffe an ihrer Oberfläche unter geeigneten Bedingungen zu immobilisieren. Zu dieser Klasse von Adsorbern zählen beispielsweise die Materialien, die in DE 10 2011 105 525 A1 beschrieben sind, insbesondere Membranen, die adsorbierende Eigenschaften haben und die sich in ihren Eigenschaften an der äußeren beziehungsweise inneren Oberfläche nicht von den Eigenschaften im Volumen unterscheiden. Darüber hinaus stellen Materialien wie Diatomeenerde, Silicagel, Kieselgur, Aktivkohle und mineralische Erden, nanostrukturierte Sorptionsmaterialien und Erden, wie in US 2010/0100027 A1 beschrieben, Adsorber im Sinne dieser Erfindung dar.

In bevorzugen Ausführungsformen der vorliegenden Erfindung werden Adsorber verwendet, die als mechanisch integraler Formkörper vorliegen, d.h. Adsorber, die keine losen Schüttungen sind. Eine besonders bevorzugte Ausführungsform ist die Ausführung des Adsorbers als ein poröser Formkörper in Form einer Membran.

Der Prozessadsorber P des erfindungsgemäßen Verfahrens weist eine spezifische Adsorptionskapazität $k_1$ für die verblockende adsorbierende Spezies auf, liegt in einem Maßstab $M_1$ vor und weist demnach eine Gesamtadsorptionskapazität von $k_1 \times M_1$ für die verblockende adsorbierende Spezies auf. Ebenso weist der Testadsorber T eine spezifische Adsorptionskapazität $k_2$ für die verblockende adsorbierende Spezies auf, liegt in einem Maßstab $M_2$ vor und weist demnach eine Gesamtadsorptionskapazität von $k_2 \times M_2$ für die verblockende adsorbierende Spezies auf.

$$0,1 < \frac{k_1 M_1}{k_2 M_2} < 10$$

Gemäß der vorliegenden Erfindung gilt , da in diesem Fall die Gesamtadsorptionskapazitäten des Prozessadsorbers P und des Testadsorbers T für die verblockende adsorbierende Spezies annähernd gleich sind und so das Testsystem sehr realistische Aussagen über das Filtrationssystem im Produktionsmaßstab ermöglicht. Je ähnlicher sich die Gesamtadsorptionskapazitäten des Prozessadsorbers P und des Testadsorbers T sind, desto realistischer spiegelt das Testsystem das Filtrationssystem im Produktionsmaßstab wider. Demzufolge gilt noch bevor-

$$0,5 < \frac{k_1 M_1}{k_2 M_2} < 5 \qquad\qquad 0,9 < \frac{k_1 M_1}{k_2 M_2} < 1,1$$

zugter und am meisten bevorzugt .

Bindende oder immobilisierende Eigenschaften sind für das Material der Adsorber charakteristisch. Die Menge von Stoffen, die gebunden werden kann, hängt von der Verfügbarkeit von molekularen Bindungsplätzen ab. Grundlegende Arbeiten zu der Beschreibung solcher Sorptionsphänomene, wie sie hier beschrieben und verwendet werden, finden sich beispielsweise in "S. Brunauer, P. H. Emmett, E. Teller: Adsorption of Gases on Multimolecular Layers. In: J.Am.Chem.Soc. 60, Nr. 2, 1938, S. 309-319" oder in "Langmuir: Surface Chemistry. Nobel Lecture, December 14, 1932. In: Nobel Lectures, Chemistry 1922-1941. Elsevier Publishing Company, Amsterdam, 1966". Die Zahl der Bindungsstellen für ein bestimmtes Sorbens wird makroskopisch als die aufgenommene, also adsorbierte oder immobilisierte Masse eines Sorbens beschrieben (Kapazität). Dabei kann die Kapazität auch auf die Masse oder das Volumen des Adsorbers normiert sein, um zu einer maßstabsunabhängigen Materialkontante, einer spezifischen Kapazität, zu gelangen, da die Zahl der Bindungsstellen dazu proportional ist.

Im Sinne dieser Erfindung ist es hinreichend, die Kapazität mit einem geeigneten Modell des Sorbens zu bestimmen. Für die hier vorgestellte Methode der Skalierung ist unerheblich, ob die bestimmte Kapazität der Adsorber exakt der Menge des realen Sorbens entspricht, so lange damit ein konstanter Anteil der verfügbaren Bindungsplätze der verschiedenen Adsorber gleichen Materials abgebildet wird, also die Proportion der Kapazität zweier Adsorber aus dem gleichen Material mit unterschiedlichen Größenausschlusseigenschaften bestimmt werden kann.

Die Bestimmung der Gesamtadsorptionskapazitäten des Prozessadsorbers P und des Testadsorbers T unterliegt erfindungsgemäß keiner besonderen Einschränkung. Geeignete Methoden zur Bestimmung der Kapazität sind im Sinne dieser Erfindung beispielsweise die Bestimmung der inneren Oberfläche durch Gassorption, insbesondere gemäß einer Langmuir-Isotherme oder einer BET-Isotherme (Brunauer-Emmett-Teller-Isotherme).

Eine alternative Methode zur Bestimmung der Adsorptionskapazität ist die Bestimmung über eine Durchbruchskurve. Dabei wird der Adsorber mit einem konstanten Zustrom einer konstanten Konzentration an Sorbens als Komponente eines Gemischs verschiedener Stoffe oder als Reinstoff beaufschlagt und die Konzentration oder Menge des Sorbens sowohl vor als auch hinter dem Adsorber gemessen. Erreicht die Konzentration hinter dem Adsorber 10% der Konzentration vor dem Adsorber, ist die Kapazität der Adsorbers erschöpft und die Massendifferenz zwischen beaufschlagtem Sorbens im Zustrom und der Masse der abfließenden Sorbens im Abstrom des Adsorbers dient als Maß für die Kapazität des Adsorbers. Um kinetische Effekte zu erkennen und zu minimieren wird, die Durchbruchskurve bei verschiedenen Zustromgeschwindigkeiten (definiert als zuströmende Masse Sorbens pro Zeiteinheit) mit Unterschied um mindestens einen Faktor 5 bestimmt. Die Zustromgeschwindigkeit wird dann reduziert, bis die Kapazität unabhängig von der Geschwindigkeit des Zustroms wird. Für den Fachmann ist es ersichtlich, dass es auch hinreichend ist, die dynamische Kapazität für die tatsächlich verwendeten Prozessparameter bei den jeweiligen Maßstäben zu bestimmen, die skaliert werden sollen. Die Methode zur Bestimmung der Konzentration des Sorbens erfolgt je nach Natur des Sorbens. Dem Fachmann sind die notwendigen Methoden bekannt, daher wird hier nur beispielhaft auf eine nicht erschöpfende Auswahl gängiger Methoden verwiesen: gängige Methoden sind absorptions- oder fluoreszenzbasierte Nachweise im Bereich IR, sichtbares Licht, UV; Nachweise über Brechungsindex, elektrochemisches Potential an geeigneten Elektroden; Nachweise über Leitfähigkeit, spezifische Wärme, Dielektrizität und andere Methoden, welche die Zusammensetzung der sorbenshaltigen Gemische und die Konzentration des Sorbens berücksichtigen.

In einer Ausführungsform der vorliegenden Erfindung können auch Bindungsversuche mit Modellproteinen verwendet werden, um die Kapazität zu bestimmen. Eine gängige Methode zur Bestimmung der Proteinbindung ist dabei der BCA-Test ("BCA" entspricht 2,2-Bichinolin-4,4-dicarbonsäuredinatriumsalz-dihydrat).

Eine weitere Methode ist die statische Inkubation, also ein Inkontaktbringen des Sorbens mit dem Adsorber und eine nachfolgende Bestimmung der Massenbilanz des ungebundenen und gebundenen Sorbens. Bei der Verwendung von Proteinen stehen beispielsweise die Bestimmung der Proteinkonzentration über Fluoreszenz der Gruppen oder die Bestimmung mittels der Absorption von UV-Licht im Bereich 270 bis 280 nm oder bei 230 nm Wellenlänge zur Verfügung. Die Methode zum Bilanzieren der Masse gebundener oder nicht gebundener Sorbentien ist für den Fachmann leicht auszuwählen und richtet sich nach der Natur des Sorbens.

Gemäß einer Ausführungsform weist der Testadsorber T eine Größenausschlussgrenze (90% Cut-off) von 10 nm oder größer, bevorzugt 20 nm oder größer und besonders bevorzugt 50 nm oder größer auf, da besonders bevorzugte Testpartikel Viren mit einer Größe von 100 bis 200 nm sind.

Größenausschlussfilter im Sinne der vorliegenden Erfindung sind Mikrofilter und Ultrafilter, wie in Pure Appl. Chem., 1996, 68, 1479 definiert. Dabei ist eine entsprechende Mikrofiltration als ein druckgetriebenes Membran-basiertes Trennungsverfahren definiert, in welchem Teilchen und gelöste Makromoleküle größer als 0,1 $\mu$m zurückgehalten werden. Die Ultrafiltration wird hingegen als ein druckgetriebenes Membran-basiertes Trennungsverfahren definiert, in welchem Teilchen und gelöste Makromoleküle kleiner als 0,1 $\mu$m und größer als etwa 2 nm zurückgehalten werden. Als Material für Größenausschlussfilter kommen beispielsweise Polymere aus Cellulose, Cellulosederivaten wie -acetat und -nitrat, Polypropylen, Polyethylen, Polyetherketonen, Polyvinylidenfluorid, Polysulfonen, Polyethersulfonen, aromatischen und aliphatischen Polyamiden, Keramiken wie $Al_2O_3$, Silikate, Nitride, Boride und Mischungen dieser Stoffe in Frage, d.h. Materialien, aus denen sich poröse, mechanisch integre Formkörper herstellen lassen. Die Größenausschlussgrenze (90% Cut-off) des Größenausschlussfilters ist im erfindungsgemäßen Verfahren kleiner als die Größenausschlussgrenze (90% Cut-off) des Testadsorbers. Gemäß einer bevorzugten Ausführungsform ist die Größenausschlussgrenze (90% Cut-off) des Größenausschlussfilters kleiner als 1 $\mu$m, mehr bevorzugt kleiner als 200 nm und besonders bevorzugt von 50 nm bis 2 nm.

Die verblockende adsorbierende Spezies, vor welcher der Größenausschlussfilter G durch den seriell vorgeschalteten Prozessadsorber P bzw. Testadsorber T geschützt werden soll, unterliegt erfindungsgemäß keiner besonderen Einschränkung. Beispielsweise ist die verblockende adsorbierende Spezies mindestens ein Mitglied der Gruppe, bestehend aus Biopolymeren (beispielsweise Proteine, DNS und RNS, Bestandteile extrazelluläre Matrices wie Polysaccharide), Biopolymeraggregaten (beispielsweise Proteinaggregate, stoffreine Aggregate der vorgenannten Biopolymere und gemischte Aggregate derselben), biologischen Partikeln (beispielsweise Mikroorganismen wie Bakterien und Mykoplasmen).

Das erfindungsgemäße Verfahren kann vorteilhafterweise dazu verwendet werden, Virusabreicherungsstudien im Produktionsgroßmaßstab im Kleinmaßstab abzubilden und zu analysieren. Insbesondere erlaubt es das erfindungsgemäße Verfahren im Gegensatz zu den aus dem Stand der Technik bekannten Verfahren, quantitativ zwischen der Virusretention durch den Vorfilter und der durch den Virusfilter bewirkten Virusretention zu unterscheiden. Hierdurch wird es vorteilhafterweise möglich, sicher und präzise die Virusretention in Virusabreicherungsstudien zu bestimmen, die ausschließlich auf den Virusfilter zurückgeht, ohne dass der Wert für die Virusretention durch den Beitrag des Vorfilters verfälscht wird.

Figur 1 zeigt ein Filtrationssystem im Produktionsmaßstab, umfassend einen Prozessadsorber P und einen Größenausschlussfilter G.

Figur 2 zeigt ein Filtrationssystem im Produktionsmaßstab (links) und ein Testsystem (rechts).

Figur 3 zeigt das Ergebnis des Beispiels 1 für Goldnanopartikel.

Figur 4 zeigt das Ergebnis des Beispiels 1 für Latexbeads.

Figur 5 und Figur 6 zeigen die Ergebnisse der Filtrationen aus Beispiel 3.

Die vorliegende Erfindung wird anhand der folgenden, nicht einschränkenden Beispiele näher erläutert.

**Beispiele**

Beispiel 1: Bestimmung der Größenausschlussgrenze von Adsorbern

Als Adsorber werden in diesem Ausführungsbeispiel mikroporöse Membranen aus Polyamid verwendet. Dabei stellen "25006" und "25058" zwei Membranen dar, die aus den gleichen Grundstoffen (Basismaterial) hergestellt sind, aber unterschiedliche Größenausschlusseigenschaften besitzen: "25058" ist ein Prozessadsorber im Sinn dieser Erfindung, "25006" stellt einen Testadsorber im Sinn dieser Erfindung dar. Die vorgenannten Materialien haben folgende Eigenschaften:

25006: Polyamid-Adsorber, nominelle Porengröße 0,45 μm,
25058: Polyamid-Adsorber, nominelle Porengröße 0,10 μm.

Die Membranen werden als Filterscheiben verwendet. Als Probepartikel dienen fluoreszierende Latexbeads. Die Größe der Beads wird mit dynamischer Lichtstreuung im "backscattering modus" (Rückstreuungsmodus) aus dem z-Average ("z-Durchschnitt") bestimmt. Zur Bestimmung wird zeta sizer nano der Firma Malvern verwendet.

Danach werden die verschiedenen Adsorber zur Filtration von a) Goldnanopartikeln und b) zur Filtration fluoreszierender Latexbeads eingesetzt, um LRV$_{Testadsorber}$ und LRV$_{Prozessadsorber}$ zu bestimmen.

Darüber hinaus wird die Rückhaltung LRV$_{Testsystem}$ dieser Partikel im Testsystem aus Testadsorber T und Größenausschlussfilter G, bestimmt, wie im erfindungsgemäßen Verfahren in Schritt (b) beschrieben.

Zum Vergleich wird auch die Rückhaltung dieser Partikel im Prozessystem aus dem Prozessadsorber P und dem Größenausschlussfilter G ermittelt. Die Ermittlung von LRV$_{Größenausschlussfilter}$ aus diesem Prozesssystem im nachfolgenden Schritt (c) ist nicht erfindungsgemäß, sondern Stand der Technik. In diesem Ausführungsbeispiel werden Nachteile dieser bisher üblichen Art der Ermittlung gezeigt.

In Schritt (c) des erfindungsgemäßen Verfahrens wird die Rückhaltung des Größenausschlussfilters aus der Differenz LRV$_{Testsystem}$ - LRV$_{Testadsorber}$ bestimmt.

Bei der nicht erfindungsgemäßen Berechnung nach dem Stand der Technik, d. h. gemäß LRV$_{Größenausschlussfilter}$ = LRV$_{Prozesssystem}$ - LRV$_{Prozessadsorber}$, ergeben sich Nachteile, wie sie in der Darstellung der Ergebnisse dieses Ausführungsbeispiels weiter beschrieben werden.

Beschreibung der Bestimmung der Partikelgröße und der Filtration:
Nanopartikel stellen neben Viren eine Art von Partikeln dar, wie sie vom Größenausschlussfilter zurückgehalten werden sollen. In diesem Ausführungsbeispiel werden Nanopartikel aus Gold und fluoreszenzmarkierte Latexpartikel verwendet. Als Adsorber P und T werden Polyamid-Membranschichten (PA-Membranschichten) eingesetzt, der Größenausschlussfilter ist in diesem Fall eine Polyethersulfonmembran mit einer nominellen Porengröße von 20 nm.

Verwendet werden Partikel mit 50 nm (Gold/Nanopartz; CAS: A11-50-CIT-100; Lot: E2451C) und 200 nm (fluoreszierende Latexbeads/Thermo Fisher; Fluoro-Max; CAS: G100) nomineller Größe nach Herstellerangabe. Die Größe der Partikel dient als Modell für Viren unter Verwendung von PA-Adsorbern mit unterschiedlicher Porengröße.

Um die Monodispersität und Größe der Partikel zu verifizieren, wird sie mit Hilfe von dynamischer Lichtstreuung ermittelt.

Bestimmen der Größe der Partikel am DLS (*"Dynamic Light Scattering",* dynamische Lichtstreuung):

Beschreibung der Methode:

- Einstellungen für DLS:

    - Material Latex, beziehungsweise Gold
    - Dispersant: Wasser @25°C Haltetemp. 180 s
    - Zelltyp ZEN0040 (kleine Küvette)
    - Messung: 173° Backscatter ("Rückstreuung")
    - Datenverarbeitung: allgemeiner Zweck (normale Auflösung)

3-fach Bestimmung mit drei unabhängigen Proben der Partikellösung ergibt folgende Größen:
Goldpartikel 50 nm:

- z-Average: 51,8 ±0,7 nm

Latexbeads 200 nm:

- z-Average: 201,6 ±0,3 nm

Die Fehlerangabe bezieht sich auf die statistisch ermittelte Standardabweichung der drei Messungen.

Filtrieren und Bestimmung der Partikelkonzentrationen in Unfiltrat und Filtrat:
Der Prozessadsorber P wird durch einen Schichtaufbau aus Polyamidmembranen mit einer nominellen Porengröße von 0,1 μm repräsentiert.

Der Testadsorber T wird durch die Kombination aus zwei unterschiedlichen Membranen in einem Schichtaufbau von drei Schichten der Polyamidmembran 25005 (0,65 μm nominelle Porengröße) und einer Schicht der Membran 25006 (0,45 μm nominelle Porengröße) repräsentiert.

Gold 50 nm: Nachweis über UV-VIS

Lösung: Goldlösung mit 0,26% SDS (Natriumdodecylsulfat) mit einer optischen Dichte bei 527 nm von etwa 1 Filtration:

Die Membran wird mit 50 L/m² (Volumen pro Anströmfläche des Adsorbers), mindestens jedoch dem fünffachen Totvolumen der Filtrationsapparatur, mit einer 0,26% SDS-Lösung vorgespült.

Dann wird die Goldpartikellösung filtriert. 30L/m² der Goldpartikellösung werden über die Adsorber filtriert, mindestens jedoch das dreifache des Totvolumens der Filtrationsapparatur. Anschließend werden zwei Proben zur Bestimmung der Konzentration der Partikel separat aufgefangen:

-2 Fraktionen je 10L/m² werden getrennt voneinander aufgefangen und auf den Partikelgehalt analysiert.

Die Konzentrationsmessung der Partikel erfolgt gegen eine Kalibriergerade, die aus einer Verdünnungsreihe der oben beschriebenen Partikellösung erzeugt wird.

Gold-Nanopartikel werden über ihre Absorption bei 527 nm quantifiziert.

Die Latexbeads werden anhand ihrer Fluoreszenz quantifiziert. Dabei werden folgende Einstellungen verwendet:

Lösung 200 nm: 0,5% kommerzielle Latexbeads Lösung in RO-$H_2O$ mit 0,26% SDS

- Latex leicht schwenken, danach ca. 15 sec in ein Ultraschallbad halten.

Filtration:

- Membran mit 5 ml 0,26% SDS-Lösung spülen (Virosart® Max 0,5 bar; 25006 0,1 bar)
- 3 g mit Latexbeads Lösung spülen, dann
- 2 × 1 g Fraktionen über Waage auffangen (4 ml Glasröhrchen mit Deckel)

Messen:

- Spektrometer auf Fluoreszenz einstellen (Platte schwarz mit durchsichtigem Boden "nunc 96Well" CAS:265301)
- Exzitationswellenlänge: 468 nm; Emissionswellenlänge: 508 nm
- Standardreihe/%: 100,75,50,25,0
- Volumen zum Messen: 200 μl
- Verstärkung 200 nm: 86

*Ergebnisse Größenbestimmuna:*

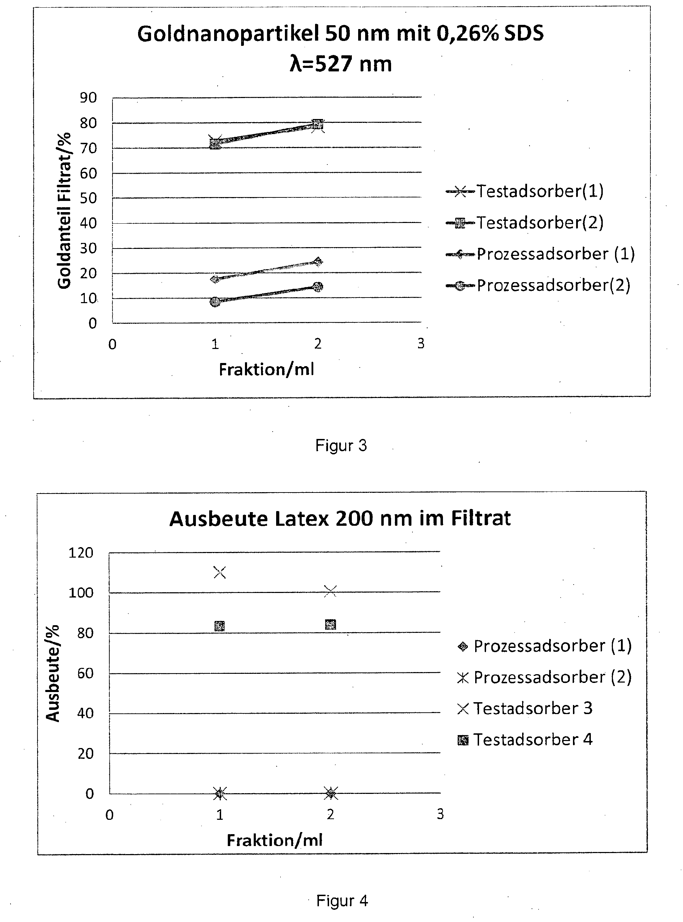

Goldfiltration (siehe Figur 3):
Der Rückhalt des Virosart® Max (25058 = Prozessadsorber) liegt im Mittel bei 80%, bei der Membran 25005 etwa bei 25% (Testadsorber). Der Rückhalt für Goldnanopartikel mit d=50 nm für die Membran 25058 entspricht einem $LRV_{Prozessadsorber} = \log(c_{feed}/c_{filtrate}) = \log (1/0,2) = 0,69$. Für den Testadsorber ergibt sich ein Rückhalt von $LRV Testadsorber = \log (c_{feed}/c_{filtrate}) = \log (1/0,75) = 0,12$.

| Modus Bestimmung der Konzentration von Gold-Nanopartikeln | Absorption |
|---|---|
| Küvette Wellenlänge | 527 nm |
| Bandbreite | 9 nm |
| Anzahl der Blitze | 25 |
| Ruhezeit | 0 ms |

Latexfiltration (siehe Figur 4):
200 nm: Der Rückhalt des Virosart® Max (25058 = Prozessadsorber) liegt im Mittel bei >99,9%, bei der Membran 25005 (Testadsorber) etwa bei 10%. Der Rückhalt für Latexbeads der Größe 193 nm liegt für den Prozessadsorber bei größer $LRV_{Prozessadsorber} = \log (c_{feed}/c_{filtrate}) = \log (1/0,001) = 3$. Für den Testadsorber ergibt sich ein $LRV_{Testadsorber} = \log (c_{feed}/c_{filtrate}) = \log (1/0,9) = 0,04$.

| Modus Bestimmung der Konzentration Latexbeads | Fluoreszenz Messung von oben |
|---|---|
| Exzitationswellenlänge | 468 nm |

(fortgesetzt)

| Modus Bestimmung der Konzentration Latexbeads | Fluoreszenz Messung von oben |
|---|---|
| Emissionswellenlänge | 508 nm |
| Exzitationsbandbreite | 9 nm |
| Emissionsbandbreite | 20 nm |
| Verstärkung | 86 Manuell |
| Anzahl der Blitze | 25 |
| Integrationszeit | 20 μs |
| Verzögerungszeit | 0 μs |
| Ruhezeit | 0 ms |
| Bereich der Platte | D6-D10; E6-H9 |

Erfindungsgemäß wird die Vorfiltration durch den Prozessadsorber bzw. den Testadsorber "in-line" durchgeführt, so dass sie der Richtlinie genügt, dass der Mechanismus der Virusabreicherung eindeutig identifiziert ist.

Die Rückhaltung der Testsysteme $LRV_{Testsystem}$ wird für die Kombination aus dem Testadsorber und als Vergleichsbeispiel auch mit dem Prozessadsorber für die Gold-Nanopartikel mit 50 nm Größe und für die Latexbeads mit einer Größe von 193 nm bestimmt. Die Konzentrationsbestimmungen der Partikel werden wie bei der Bestimmung von $LRV_{Testadsorber}$ durchgeführt.

Als Größenausschlussfilter dient in diesem Ausführungsbeispiel ein Virenfilter Virosart® CPV (Polyethersulfonmembran mit einer nominellen Porengröße von 20 nm), erhältlich bei Sartorius Stedim Biotech GmbH.

Die Rückhaltung der Gold Nanopartikel der beiden Systeme ergibt sich zu:

$LRV_{Testsystem}$(25005/25006 + Virosart® CPV) >3,2
$LRV_{Testsystem}$(25058 + Virosart® CPV) >3,2

Die Rückhaltung der Latexbeads der beiden Systeme ergibt sich zu:

$LRV_{Testsystem}$(25005/25006 + Virosart® CPV) ≥3,2
$LRV_{Testsystem}$(25058 + Virosart® CPV) ≥3,2

Analog zur Ermittlung der Rückhaltung der verwendeten Testadsorber und des Prozessadsorbers wird nun im Schritt (c) des erfindungsgemäßen Verfahrens durch Subtraktion des $LRV_{Testadsorber}$ von $LRV_{Testsystem}$ der $LRV_{Größenausschlussfilter}$ berechnet, d.h. $LRV_{Größenausschlussfilter} = LRV_{Testsystem} - LRV_{Testadsorber}$.

Für die Rückhaltung des Größenauschlussfilters Virosart® CPV ergibt sich damit für die Rückhaltung der Gold-Nanopartikel unter Verwendung des Adsorbers 25058:

$LRV_{Größenausschlussfilter} = LRV_{Testsystem} - LRV{Testadsorber}$(25058)
$LRV_{Größenausschlussfilter}$ ≥3,2 -0,69
$LRV_{Größenausschlussfilter}$ ≥ 2,51

Unter Verwendung des erfindungsgemäßen Verfahrens mit Verwendung des Adsorbers 25006/25005 ergibt sich für die Rückhaltung der Gold-Nanopartikel:

$LRV_{Größenausschlussfilter} = LRV_{Testsystem} - LRV_{Testadsorber}$(25006/25005)
$LRV_{Größenausschlussfilter}$ ≥3,2 -0,12
$LRV_{Größenausschlussfilter}$ ≥ 3,08

Analog wird nun die Rückhaltung für die Latexbeads bestimmt. Für die Verwendung des Adsorbers 25058 ergibt sich:

$LRV_{Größenausschlussfilter}$ ≥3,2 -3,0
$LRV_{Größenausschlussfilter}$ ≥0,2

Unter Verwendung des erfindungsgemäßen Verfahrens mit dem Adsorber 25006/25005 wird eine höhere Rückhaltung nachgewiesen:

$LRV_{Größenausschlussfilter} \geq 3,2 -0,04$

$LRV_{Größenausschlussfilter} \geq 3, 16$

Beispiel 2: Bestimmung der Adsorptionskapazität der Adsorber von Beispiel 1

Die Kapazität wird anhand der statischen Proteinadsorption mittels BCA-Reagenz ermittelt. Die Redox-Reaktion des BCA Reagenz dient zum Nachweis von Protein. Die Färbung von Lösungen des BCA Reagenz wird als Absorbanz bei 562 nm quantifiziert. Eine Kalibriergerade aus bekannten Proteinkonzentrationen wird erstellt und die Messwerte mit dieser Kalibrierung ausgewertet.

Detaillierte Beschreibung:

1. Je Petrischale 3 ml $\gamma$-Globulin-Lösung (3 mg/ml) zugeben.

2. Die einzelnen Testproben (13 mm Ø) und Referenzproben mit der Pinzette in jeweils eine entsprechend beschriftete Petrischale (3,5 cm) geben, wobei jeglicher direkter Handkontakt zu vermeiden ist. (Eine direkte Kennzeichnung der Proben kann - falls erforderlich - mit einem Elektrophoresekugelschreiber vorgenommen werden).

3. Die Proben im Schüttelapparat (80/min) 3 h bei Raumtemperatur (ca. 20°C) inkubieren.

4. Die Proben mit Pinzette aus den Petrischalen entnehmen und in entsprechend beschriftete Petrischalen (10 cm) umsetzen.

5. Je Petrischale 15 ml 0,05 M KPi-Puffer pH 7,0 zugeben und 15 min schütteln, danach Pufferlösung mit Wasserstrahlpumpe vorsichtig absaugen und diesen Vorgang noch 3 x wiederholen.

6. Die Filterproben einzeln in entsprechend beschriftete Petrischalen (3,5 cm) umsetzen.

7. Zur Erstellung der Eichkurve jeweils 3 Proben zu je 25, 50, 75, 100 $\mu$l (Eichproben) und 0 $\mu$l (=Blindprobe) $\gamma$-Globulin-Lösung (1 $\mu$g/$\mu$l) einzeln in entsprechend gekennzeichnete Petrischalen (3,5 cm) pipettieren, je 2000 $\mu$l BCA-Reagens zufügen und sofort auf den Schütteltisch stellen, anschließend die einzelnen Membranproben in gleicher Weise mit je 2000 $\mu$l BCA-Reagens versehen und bei Raumtemperatur (ca. 20°C) schütteln.

8. Die photometrische Vermessung der Proben erfolgt in 1 cm-Halbmikroküvetten. Nach einer Stunde wird zuerst der Extinktionswert der Blindprobe bei 562 nm bestimmt, für Kontrollzwecke notiert und für die nachfolgenden Messungen auf Null gesetzt. Anschließend werden die Eichlösungen und Membranproben sukzessive ohne Unterbrechung und in der Reihenfolge ihrer Präparation gemessen und die Extinktionswerte im Prüfprotokoll notiert.

Die so ermittelten Adsorptionskapazitäten werden zur Bestimmung der Menge der Adsorber, hier als Flächenverhältnisse relativ zum Prozessadsorber, verwendet. Die Ergebnisse der Kapazitätsbestimmung und die resultierenden Mengen der entsprechenden Adsorber werden in Tabelle 1 zusammengefasst:

Tabelle 1: Bestimmung der Bindung von IgG bei pH=6 mit verschiedenen Adsorbern mit BCA-Test (Spalte:BCA_IVIG) und Darstellung der Skalierungsfaktoren zwischen Prozessadsorbern und Testadsorbern (Spalte: Flächenverhältnis nach BCA).

| Kategorie | Typ | Porengröße (nominal [$\mu$m] | BCA_IVIG [$\mu$g, cm$^2$] | Flächenverhältniss nach BCA | Standzeit IVIG HC als Endfilter [l/m$^2$ in 4h] |
|---|---|---|---|---|---|
| | Polyamid Adsorber | | | | |
| Testadsorber | 25004 | 0,8 | 52 | 1,62 | 89 |
| Testadsorber | 25005 | 0,65 | 59 | 1,42 | 91 |
| Testadsorber | 25006 | 0,45 | 71 | 1,18 | 83 |
| Prozessadsorber | 25058-SF | 0,1 | 84 | 1 | 84 |
| | | | pH=6 | | pH=6 |

(fortgesetzt)

| | Negative Ladung: starke Kationen Austauscher (AMPS-MBAm) | | | | |
|---|---|---|---|---|---|
| Testadsorber | modif. 15445 | | 52 | 1,62 | 200 |
| Prozessadsorber | modif. 15458_3/0,6 | | 87 | 0,97 | 165 |
| Prozessadsorber | modif. 15458_2/0,4 | | 60 | 1,40 | 134 |
| Referenz: ohne Ladungsmodifizierung | 15458 | 0,1 | 3,3 | 25,45 | |
| | 25058 | | | | 147 |
| AMPS : 2-Acrylamido-2-methylpropansulfonsäure MBAm: N,N'-Methylenbisacrylamid | | | | | |

Beispiel 3: Skalierung eines Testadsorbers, der eine abweichende Größenausschlussgrenze zum Prozessadsorber aufweist.

In der vorstehenden Tabelle 1 sind die Skalierungsfaktoren, die sich aus den Bindungskapazitäten des BCA-Tests ergeben, bereits dargestellt. Die praktische Anwendung dieser Faktoren wird im Folgenden beschrieben.

*Filtration einer humanen IgG-Proteinlösung über verschiedene Polyamidvorfilter:*

Zur Filtration wurde eine Lösung eines humanen IgG (5%ige Lösung, SeraCare, Katalog- Nr. HS-475-1L) verwendet, die mit einer 50 mM KPi-Pufferlösung, pH 7,2, auf eine Konzentration von 0,5% verdünnt wurde.

Verwendete Membranen:

Vorfilter $(VF)_1$: Polyamid 6

- nominale Porengröße: 0,35 $\mu$m
- Durchflusszeit für Wasser [ml/ (min cm$^2$ bar)]: 23
- Bubble Point ("Blasenpunkt") mit Wasser, visuell [bar]: 2,9

Vorfilter $(VF)_2$: Polyamid 6

- nominale Porengröße: 0,1 $\mu$m,
- Durchflusszeit für Wasser [ml/ (min cm$^2$ bar)]: 6
- Bubble Point mit Wasser, visuell [bar]: 7

Endfilter (EF): oberflächenmodifizierte, virusretentive Polyethersulfon Virosart$^®$HC Membran (oberflächenmodifizierte Polyethersulfonmembran mit einer nominellen Porengröße von 20 nm), doppellagig

- nominale Porengröße: 20 nm
- Durchflusszeit für Wasser [ml/ (min cm$^2$ 2bar)]: 0,19
- effektive Anströmfläche: 4,7 cm$^2$

Beide Filtrationen wurden "in-line" betrieben, d.h. VF und EF wurden vor Filtrationsbeginn über den Luer-Anschluss miteinander verbunden. Das $VF_1$-Modul hat eine effektive Anströmfläche von 4,7 cm$^2$, während das $VF_2$-Modul eine effektive Anströmfläche von 3,11 cm$^2$ hat.

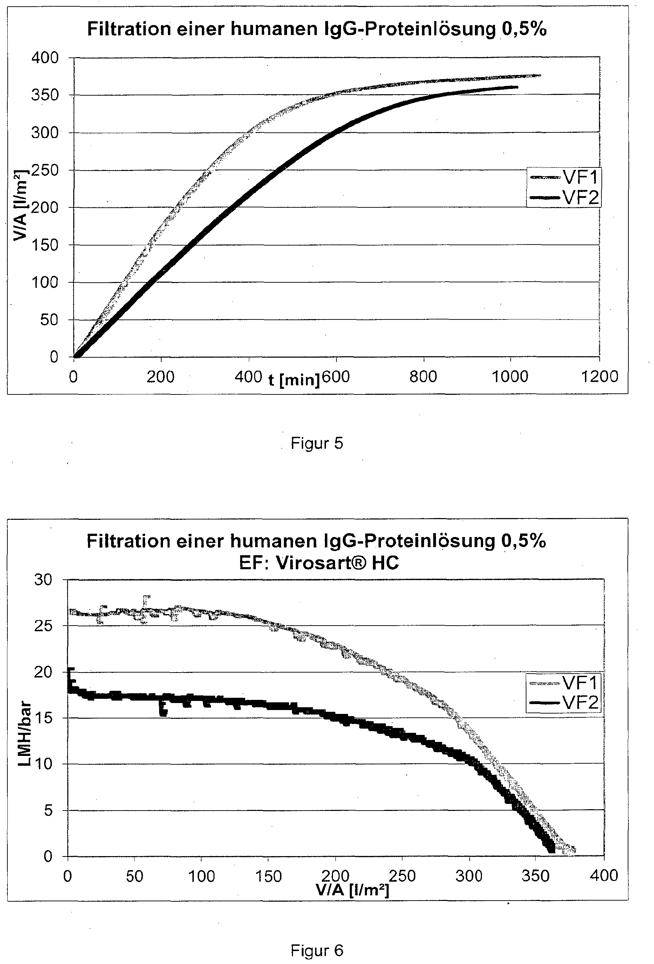

Die Filtrationen wurden parallel mit der gleichen Stammlösung und unter einem Druck von 2 bar durchgeführt. Die unterschiedlichen Steigungen in den Figuren 5 und 6 sind durch den unterschiedlichen Flusswiderstand der beiden Adsorber bedingt.

Figuren 5 und 6 zeigen, dass das filtrierte Volumen bei beiden Filtrationen nahezu identisch ist, weil die verwendete Menge des Adsorbers, hier bezeichnet als VF1 und VF2 so skaliert wurde, dass die gleiche Bindungskapazität für Verunreinigungen erreicht wurde.

Durch die passende Skalierung der Membranflächen der Vorfilter wurde eine annähernd gleiche Standzeit erreicht.

*Auswahlkriterien zur Flächenskalierung der Vorfilter:*

Eine Möglichkeit, um die unspezifische Proteinadsorption einer Membran zu messen, findet man in der BCA-Methode. Bei dieser Methode adsorbiert das Protein an einer Membran und wird mittels BCA-Reagenz (2,2-Bichinolin-4,4-dicarbonsäuredinatriumsalz-dihydrat) photometrisch quantitativ bestimmt.

Folgende Ergebnisse sind dabei ermittelt worden:

$VF_1$: 52,7 $\mu$g/cm$^2$
VF2: 84,2 $\mu$g/cm$^2$

Um nun auf eine gleiche Adsorption während der Filtration zu gelangen, musste die Fläche des $VF_1$ um den Faktor 1,6 vergrößert werden.

## Patentansprüche

1. Verfahren zur Bestimmung des logarithmischen Reduktionswertes $LRV_{Größenausschlussfilter}$ eines Größenausschlussfilters G für ein zu klärendes Partikel einer Prozesslösung in einem Filtrationssystem, das den Größenausschlussfilters G und einen Prozessadsorber P umfasst,

   wobei der Größenausschlussfilter G durch den seriell vorgeschalteten Prozessadsorber P vor einer verblockenden adsorbierenden Spezies, enthalten in der Prozesslösung, geschützt wird und wobei durch den Prozessadsorber P das zu klärende Partikel mit einem $LRV_{Prozessadsorber}$ von 0,5 oder mehr zurückgehalten wird, umfassend die Schritte:

   (a) Bereitstellen eines Testsystems, umfassend

   einen Größenausschlussfilter G, dessen $LRV_{Größenausschlussfilter}$ zu bestimmen ist, und einen dem Größenausschlussfilter G seriell vorgeschalteten Testadsorber T, welcher aus einem gleichartigen Material wie der Prozessadsorber P besteht und durch den das zu klärende Partikel mit einem bekannten $LRV_{Testadsorber}$ von 2 oder weniger zurückgehalten wird, wobei $LRV_{Testadsorber} <$ $LRV_{Prozessadsorber}$ gilt;

   (b) Bestimmung von $LRV_{Testsystem}$ des Testsystems für das zu klärende Partikel in der Prozesslösung; und
   (c) Berechnung von $LRV_{Größenausschlussfilter}$ durch Subtraktion des $LRV_{Testadsorber}$ von $LRV_{Testsystem}$,

$$LRV = \log \frac{C_{feed}}{C_{filtrate}}$$

   wobei                               gilt,
   wobei $c_{feed}$ die Konzentration des zu klärenden Partikels in der Prozesslösung vor Durchlauf eines Filters ist und $c_{filtrate}$ die Konzentration des zu klärenden Partikels in der Prozesslösung nach Durchlauf des Filters ist, und wobei der Prozessadsorber P eine spezifische Adsorptionskapazität $k_1$ für die verblockende adsorbierende Spezies aufweist, in einem Maßstab $M_1$ vorliegt und eine Gesamtadsorptionskapazität für die verblockende adsorbierende Spezies von $k_1 \times M_1$ aufweist,
   wobei der Testadsorber T eine spezifische Adsorptionskapazität $k_2$ für die verblockende adsorbierende Spezies aufweist, in einem Maßstab $M_2$ vorliegt und eine Gesamtadsorptionskapazität für die verblockende adsorbie-rende Spezies von $k_2 \times M_2$ aufweist, und

$$0,1 < \frac{k_1 M_1}{k_2 M_2} < 10$$

   wobei                gilt.

2. Verfahren nach Anspruch 1, wobei der Testadsorber T einen $LRV_{Testadsorber}$ von 1 oder weniger aufweist.

**3.** Verfahren nach Anspruch 1 oder 2, wobei $0,5 < \dfrac{k_1 M_1}{k_2 M_2} < 5$ gilt.

**4.** Verfahren nach einem der Ansprüche 1 bis 3, wobei $0,9 < \dfrac{k_1 M_1}{k_2 M_2} < 1,1$ gilt.

**5.** Verfahren nach einem der Ansprüche 1 bis 4, wobei die Gesamtadsorptionskapazitäten des Prozessadsorbers P und des Testadsorbers T durch Gassorptionsmessung, durch Bestimmung von Durchbruchskurven, durch Bindungsversuche mit Modellproteinen oder durch statische Inkubation bestimmt werden.

**6.** Verfahren nach einem der Ansprüche 1 bis 5, wobei der Prozessadsorber P und der Testadsorber T als mechanisch integrale Formkörper vorliegen.

**7.** Verfahren nach Anspruch 6, wobei der Prozessadsorber (P) und der Testadsorber T in Form von Membranen vorliegen.

**8.** Verfahren nach einem der Ansprüche 1 bis 7, wobei die verblockende adsorbierende Spezies mindestens ein Mitglied der Gruppe, bestehend aus Biopolymeren, Biopolymeraggregaten, biologischen Partikeln, viralen Vektoren, Viren und Mikroorganismen ist.

**Claims**

**1.** A method for determining the logarithmic reduction value of a $LRV_{\text{Size exclusion filter}}$ of a size exclusion filter G for a particle that is contained in a process solution and is to be filtered out in a filtration system, which comprises the size exclusion filter G and a process adsorber P,

wherein the size exclusion filter G is protected from a blocking adsorbing species contained in the process solution by means of the process adsorber P that is connected in series upstream of the size exclusion filter and wherein the particle to be filtered out by means of the process adsorber P is retained with a $LRV_{\text{Process adsorber}}$ of 0.5 or more, comprising the steps:

(a) providing a test system, comprising a size exclusion filter G, whose $LRF_{\text{Size exclusion filter}}$ is to be determined, and a test adsorber T connected upstream of the size exclusion filter G, which consists of a material similar to the process adsorber P and by means of which the particle to be filtered out is retained with a known $LRV_{\text{Test adsorber}}$ of 2 or less, wherein $LRV_{\text{Test adsorber}} < LRV_{\text{Process adsorber}}$;
(b) determining $LRV_{\text{Test system}}$ of the test system for the particle to be filtered out in the process solution; and
(c) calculating $LRV_{\text{Size exclusion filter}}$ by subtracting the $LRV_{\text{Test adsorber}}$ from $LRV_{\text{Test system}}$,

where $LRV = \log \dfrac{c_{feed}}{c_{filtrate}}$

wherein $c_{\text{feed}}$ is the concentration of the particle to be filtered out in the process solution prior to passage of a filter and $c_{\text{filtrate}}$ is the concentration of the particle to be filtered out in the process solution after passage of the filter, and
wherein the process adsorber P has a specific adsorption capacity $k_1$ for the blocking adsorbing species, is present on a scale $M_1$ and has a total adsorption capacity for the blocking adsorbing species of $k_1 \times M_1$, and
wherein the test adsorber T has a specific adsorption capacity $k_2$ for the blocking adsorbing species, is present on a scale $M_2$ and has a total adsorption capacity for the blocking adsorbing species of $k_2 \times M_2$, and

where $0,1 < \dfrac{k_1 M_1}{k_2 M_2} < 10$ .

**2.** The method according to claim 1, wherein the test adsorber T has a $LRV_{\text{Test adsorber}}$ of 1 or less.

**3.** The method according to claim 1 or 2, where $0,5 < \dfrac{k_1 M_1}{k_2 M_2} < 5$ .

**4.** The method according to any of claims 1 to 3, where $0,9 < \dfrac{k_1 M_1}{k_2 M_2} < 1,1$ .

**5.** The method according to any of claims 1 to 4, wherein the total adsorption capacities of the process adsorber P and of the test adsorber T are determined by gas sorption measurement, by determining breakthrough curves, by binding experiments with model proteins or by static incubation.

**6.** The method according to any of claims 1 to 5, wherein the process adsorber P and the test adsorber T are present as mechanical integral shaped bodies.

**7.** The method according to any of claim 6, wherein the process adsorber (P) and the test adsorber T are present in the form of membranes.

**8.** The method according to any of claims 1 to 7, wherein the blocking adsorbing species is at least a member of the group consisting of biopolymers, biopolymer aggregates, biological particles, viral vectors, viruses and microorganisms.

**Revendications**

**1.** Procédé de détermination de la valeur de réduction logarithmique LRV$_{\text{filtre à exclusion de taille}}$ d'un filtre à exclusion de taille G pour une particule à clarifier d'une solution de processus dans un système de filtration qui comprend le filtre à exclusion de taille G et un adsorbeur de processus P,

dans lequel le filtre à exclusion de taille G est protégé par l'adsorbeur de processus P monté en série en amont d'une espèce adsorbante bloquante contenue dans la solution de processus et dans lequel la particule à clarifier est retenue par l'adsorbeur de processus P avec une LRV$_{\text{adsorbeur de processus}}$ de 0,5 ou plus, qui comprend les étapes consistant à :

a) fournir un système de test, qui comprend

un filtre à exclusion de taille G dont la LRV$_{\text{filtre à exclusion de taille}}$ est à déterminer, et
un adsorbeur de test T monté en série en amont du filtre à exclusion de taille G, lequel adsorbeur de test se compose d'un matériau similaire à celui de l'adsorbeur de processus P et par lequel la particule à clarifier est retenue avec une LRV$_{\text{adsorbeur de}}$ test connue de 2 ou moins, dans lequel LRV$_{\text{adsorbeur de}}$ test < LRV$_{\text{adsorbeur}}$ de processus ;

b) déterminer la LRV$_{\text{système de test}}$ du système de test pour la particule à clarifier dans la solution de processus ; et
c) calculer la LRV$_{\text{filtre à exclusion de taille}}$ en soustrayant la LRV$_{\text{adsorbeur}}$ de test de la LRV$_{\text{système}}$ de test,

dans lequel $LRV = \log \dfrac{C_{feed}}{C_{filtrate}}$ ,

dans lequel C$_{\text{feed}}$ est la concentration de la particule à clarifier dans la solution de processus avant la traversée d'un filtre et C$_{\text{filtrate}}$ est la concentration de la particule à clarifier dans la solution de processus après la traversée du filtre, et
dans lequel l'adsorbeur de processus P présente une capacité d'adsorption spécifique $k_1$ pour l'espèce adsorbante bloquante, existe à une échelle $M_1$ et présente une capacité d'adsorption totale pour l'espèce adsorbante bloquante de $k_1 \times M_1$,
dans lequel l'adsorbeur de test T présente une capacité d'adsorption spécifique $k_2$ pour l'espèce adsorbante bloquante, existe à une échelle $M_2$ et présente une capacité d'adsorption totale pour l'espèce adsorbante bloquante de $k_2 \times M_2$, et

dans lequel $\quad 0{,}1 < \dfrac{k_1 M_1}{k_2 M_2} < 10$ .

2. Procédé selon la revendication 1, dans lequel l'adsorbeur de test T présente une LRV$_{\text{adsorbeur de}}$ test de 1 ou moins.

3. Procédé selon la revendication 1 ou 2, dans lequel $\quad 0{,}5 < \dfrac{k_1 M_1}{k_2 M_2} < 5$ .

4. Procédé selon l'une des revendications 1 à 3, dans lequel $\quad 0{,}9 < \dfrac{k_1 M_1}{k_2 M_2} < 1{,}1$ .

5. Procédé selon l'une des revendications 1 à 4, dans lequel les capacités d'adsorption totales de l'adsorbeur de processus P et de l'adsorbeur de test T sont déterminées par la mesure de sorption du gaz, par détermination de courbes de percée, par des essais de liaison avec des protéines modèles ou par incubation statique.

6. Procédé selon l'une des revendications 1 à 5, dans lequel l'adsorbeur de processus P et l'adsorbeur de test T existent en tant que corps moulés mécaniquement intégraux.

7. Procédé selon la revendication 6, dans lequel l'adsorbeur de processus (P) et l'adsorbeur de test T existent sous forme de membranes.

8. Procédé selon l'une des revendications 1 à 7, dans lequel l'espèce adsorbante bloquante est au moins un membre du groupe composé de biopolymères, d'agrégats de biopolymères, de particules biologiques, de vecteurs viraux, de virus et de micro-organismes.

adsorbierende verblockende Spezies

Y   Zielmolekül

zu klärendes Partikel

Prozessadsorber P

Größenausschlussfilter G

Figur 1

Filtrationssystem im
Produktionsmaßstab

Testsystem

adsorbierende verblockende Spezies

Y    Zielmolekül

zu klärendes Partikel

Prozessadsorber P
bzw.
Testadsorber T

Größenausschlussfilter G

Figur 2

Figur 3

Figur 4

**Filtration einer humanen IgG-Proteinlösung 0,5%**

Figur 5

**Filtration einer humanen IgG-Proteinlösung 0,5%
EF: Virosart® HC**

Figur 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2007039069 A
- DE 102011105525 A1
- US 7118675 B2
- US 2012088228 A1
- US 20100100027 A1

**In der Beschreibung aufgeführte Nicht-Patentliteratur**

- **S. BRUNAUER ; P. H. EMMETT ; E. TELLER.** Adsorption of Gases on Multimolecular Layers. *J.Am.Chem.Soc.,* 1938, vol. 60 (2), 309-319
- Langmuir: Surface Chemistry. *Nobel Lecture,* 14. Dezember 1932
- Nobel Lectures, Chemistry. Elsevier Publishing Company, 1966
- *Pure Appl. Chem.,* 1996, vol. 68, 1479